# EUROPEAN PATENT APPLICATION

(11) **EP 2 921 113 A1**
(43) Date of publication of application: **23.09.2015**
(21) Application number: 12888501.9
(22) Date of filing: 15.11.2012
(51) Int. Cl.: A61B 6/00

(54) **SYSTEM AND APPARATUS OF DIGITAL MEDICAL IMAGE FOR X-RAY SYSTEM**

(71) Applicant: Seen, Dong June, Seoul 120-843 (KR)
(72) Inventor: Seen, Dong June, Seoul 120-843 (KR)
(74) Representative: Nederlandsch Octrooibureau
(86) International application number: PCT/KR2012/009630
(87) International publication number: WO 2014/077427

(57) **Abstract**

Digital radiography system and device for an X-ray system are provided. The digital radiography system includes: an X-ray detector that receives X-rays radiated from a generator of an X-ray system and that forms an X-ray image; a camera that captures an image of one side surface of a controller outputting a ready signal and a shot signal to control operation of the generator; and a monitoring device that analyzes image information transmitted from the camera to recognize a control signal output from the controller to the generator and that provides information on the control signal to the X-ray detector. By acquiring a control signal of the generator using an external image of the controller using the camera, it is not necessary to connect a cable for allowing the monitoring device and the X-ray detector to directly receive the control signal from the controller and thus to improve convenience of installation.

## Description

### TECHNICAL FIELD

The present invention relates to digital radiography (DR) system and device, and more particularly, to digital radiography system and device for an X-ray system, which recognizes and processes an operation of an X-ray generator controller of the X-ray system using a camera.

### BACKGROUND ART

With recent advancement of scientific technology and medical technology, the field of radiology-related equipment has rapidly grown as diagnostic equipment and radiological examinations have been diagnostic methods which are generalized to most patients. An example of diagnostic equipment which has been generally popularized as radiology-related equipment is an X-ray system. The X-ray system is used to visualize an anatomical structure and to detect presence of a pathological phenomenon, a disease, or an abnormal anatomical structure.

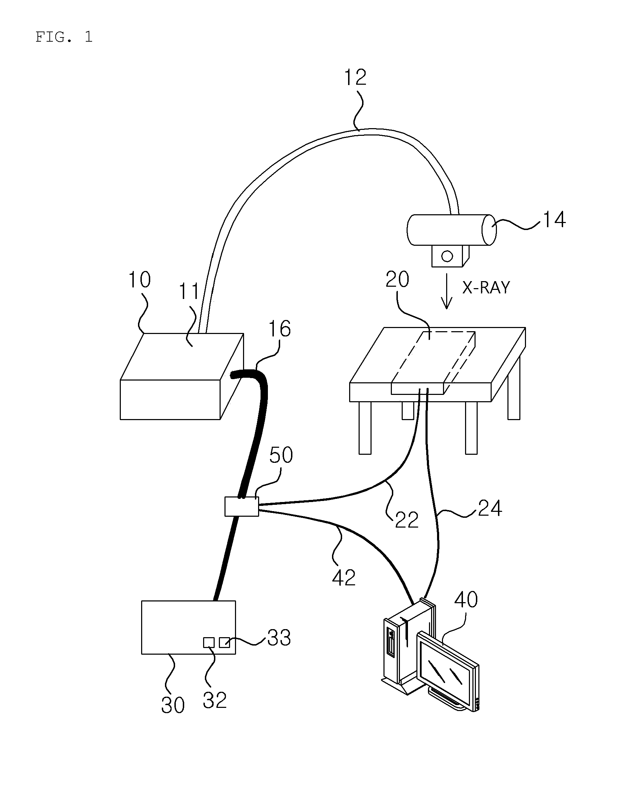

FIG. 1 is a diagram schematically illustrating a digital radiography system for an X-ray system according to the related art.

Referring to FIG. 1, in general, an X-ray system includes a generator 10 and a controller 30. A digital radiography system for acquiring an X-ray image from the X-ray system includes an X-ray detector 20, a monitoring device 40, and a bridge device 50.

The generator 10 radiates X-rays, and the X-ray detector 20 receives X-rays which are radiated from the generator 10 and transmitted by a subject and generates an X-ray image. In general, the generator 10 include a body section 11 generating X-rays and a radiation section 14 radiating X-rays, and the body section 11 and the radiation section 14 are connected to each other via a tube 12 through which X-rays pass.

The controller 30 serves to control driving of the generator 10. Examples of a control signal include a ready signal and a shot signal.

The generator 10 starts preparation (generation of X-rays and the like) for radiating X-rays when the ready signal is input, and radiates the prepared X-rays when the shot signal is input.

The control signal also has to be transmitted to the X-ray detector 20 such that X-ray detector 20 prepares for generating an X-ray image using X-rays radiated from the generator 10. The control signal also has to be transmitted to the monitoring device 40 such that the monitoring device 40 receives an X-ray image from the X-ray detector 20 via a communication cable 24. For this purpose, in the digital radiography system according to the related art, the control signal is transmitted to the X-ray detector 20 and the monitoring device 40 via the cables 22 and 42 by the bridge device 50 which is disposed in a connector 16 connected to transmit the control signal between the controller 30 and the generator 10.

However, in hospitals employing the X-ray system and the digital radiography system, or the like, the controller 30 and the monitoring device 40 are generally located in a place separated from the place in which the generator 10 and the X-ray detector 20 are located. For example, in a hospital in which radiological treatment is carried out, the generator 10 and the X-ray detector 20 are installed in a place in which a patient is present, and the controller 30 and the monitoring device 40 are installed in a room in which interested persons of the hospital is located. Accordingly, it is not easy to install the cables 22 and 42 for transmitting the control signal output from the controller 30 to the generator 10 to the X-ray detector 20 and/or the monitoring device 40. In addition, different companies often produce and deliver the X-ray system and the digital radiography system, and thus it is difficult to install a particular bridge device and the cables 22 and 24. The types of control signals used differ depending on X-ray systems of makers and thus it is not easy to install a digital radiography system suitable for an X-ray system and software thereof.

In the related art, since the monitoring device 40 receives only the control signals of the ready signal and the shot signal of the controller 30, the monitoring device 40 can monitor only the control signals and X-ray image information transmitted from the X-ray detector 20 and cannot monitor particular X-ray radiation information (such as intensity, radiation dose, and radiation period of X-rays.

### SUMMARY OF THE INVENTION

### Technical Problem

Therefore, the present invention is made to solve the above-mentioned problems and an object thereof is to provide digital radiography system and device for an X-ray system which can allow a monitoring device and an X-ray detector to acquire a control signal from a controller by acquiring an external image of the controller without using a cable for connection to the controller.

Another object of the present invention is to provide radiography system and device for an X-ray system which can easily recognize a control signal regardless of a maker of a controller by recognizing the control signal using an image of a controller display instead of directly analyzing the control signal transmitted from the controller.

Still another object of the present invention is to provide radiography system and device for an X-ray system which can acquire and monitor radiation information such as intensity, radiation dose, and radiation period of radiated X-rays using an external image of a controller.

Other objects of the present invention will become more apparent from exemplary embodiments to be described later.

### Solution to Problem

According to an aspect of the present invention, there is provided a digital radiography system including: an X-ray detector that receives X-rays radiated from a generator of an X-ray system and that forms an X-ray image; a camera that captures an image of one side surface of a controller outputting a ready signal and a shot signal to control operation of the generator; and a monitoring device that analyzes image information transmitted from the camera to recognize a control signal output from the controller to the generator and that provides information on the control signal to the X-ray detector.

Here, the controller may include display means for notifying that the ready signal or the shot signal is output, and the monitoring device may recognize the ready signal or the shot signal using the image information captured to include the display means in an image.

The display means of the controller may output information on at least one of intensity, radiation dose, and radiation period of X-rays radiated from the generator, and the monitoring device may recognize and output the information on at least one of intensity, radiation dose, and radiation period of the X-rays using the image information captured to include the display means in the image.

The camera may include a connector which is detachably attached to the controller.

According to another aspect of the present invention, there is provided a digital radiography system including: an X-ray detector that receives X-rays radiated from a generator of an X-ray system and that forms an X-ray image; an optical sensor that receives light emitted from a controller outputting a ready signal and a shot signal to control operation of the generator; and a monitoring device that analyzes information transmitted from the optical sensor to recognize a control signal output from the controller to the generator and that provides information on the control signal to the X-ray detector.

According to still another aspect of the present invention, there is provided an X-ray monitoring device including: a communication module that receives image information from a camera module capturing an image of one side of a controller which controls driving of a generator radiating X-rays to an X-ray detector forming an X-ray image; a computing module that analyzes the image information to recognize a control signal based on a ready signal or a shot signal output from the controller to the generator; and a communication interface that is connected to a communication cable for transmitting information on the control signal to the X-ray detector.

Here, the controller may include display means for notifying that the ready signal or the shot signal is output, and the computing module may recognize the ready signal or the shot signal using the image information captured to include the display means in an image.

The display means of the controller may output information on at least one of intensity, radiation dose, and radiation period of X-rays radiated from the generator, and the computing module may recognize and output the information on at least one of intensity, radiation dose, and radiation period of the X-rays using the image information captured to include the display means in the image.

### Advantageous Effects of the Invention

In the digital radiography system and device according to the present invention, by acquiring a control signal of the generator using an external image of the controller using the camera, it is not necessary to connect a cable for allowing the monitoring device and the X-ray detector to directly receive the control signal from the controller and thus to improve convenience of installation.

According to the present invention, it is also possible to easily recognize a control signal regardless of a maker of a controller by recognizing the control signal using an image of a controller display instead of directly analyzing the control signal transmitted from the controller.

According to the present invention, it is possible to further acquire and monitor radiation information such as radiation dose, intensity, and radiation time of radiated X-rays by only installing a camera for capturing an external image of the controller without changing the controller and the generator in the related art.

### BRIEF DESCRIPTION OF THE DRAWINGS

FIG. 1 is a diagram schematically illustrating a digital radiography system for an X-ray system according to the related art.
FIG. 2 is a diagram illustrating a digital radiography system for an X-ray system according to an embodiment of the present invention.
FIG. 3 is a front view of a conventional controller.
FIGS. 4 and 5 are diagrams illustrating a camera attached to one side of a controller using a connector according to an embodiment of the present invention.
FIG. 6 is a block diagram illustrating a configuration of a monitoring device according to an embodiment of the present invention.
FIG. 7 is a diagram illustrating an example in which plural cameras capturing an image of a controller is provided according to another embodiment of the present invention.
FIG. 8 is a diagram illustrating an example in which a sensor for sensing a controller is provided according to another embodiment of the present invention.

### DESCRIPTION OF EMBODIMENTS OF THE INVENTION

The invention can be modified in various forms and can have various embodiments. Specific embodiments will be illustrated in the drawings and described in detail. However, the embodiments are not intended to limit the invention, but it should be understood that the invention includes all modifications, equivalents, and replacements belonging to the concept and the technical scope of the invention.

Hereinafter, embodiments of the invention will be described in detail with reference to the accompanying drawings. In describing the invention with reference to the accompanying drawings, like elements are referenced by like reference numerals or signs regardless of the drawing numbers and description thereof is not repeated.

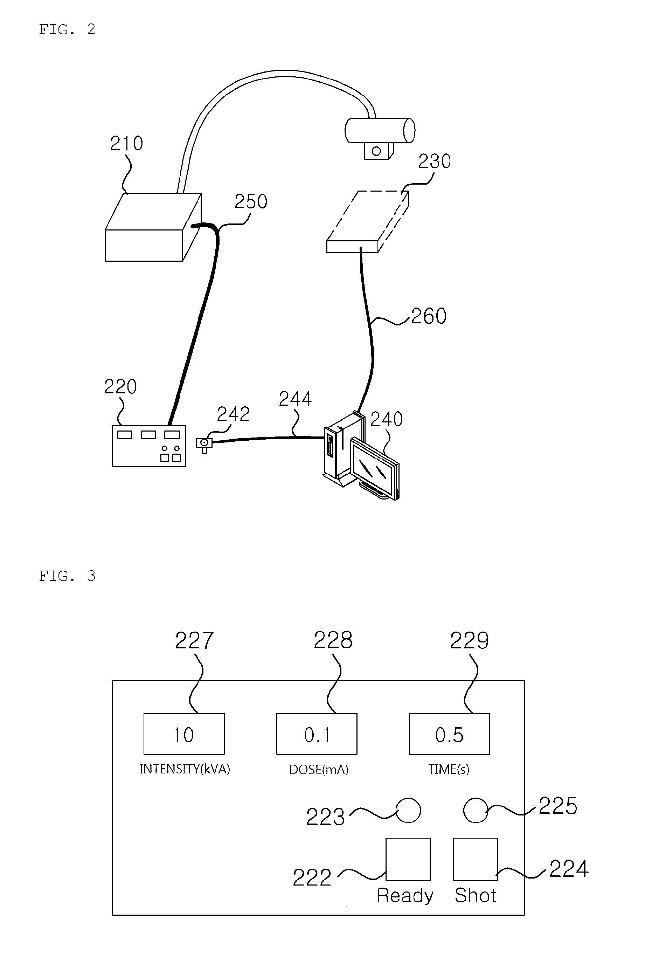

FIG. 2 is a diagram illustrating a digital radiography system for an X-ray system according to an embodiment of the present invention. FIG. 3 is a front view of a conventional controller.

Referring to FIG. 2, the digital radiography system for an X-ray system according to an embodiment includes an X-ray detector 230, a monitoring device 240, and a camera 242.

The camera 242 captures an image of one side of a controller 220 of the X-ray system and transmits the captured image information to the monitoring device 240. In this embodiment, only one camera is provided, but two or more cameras may be provided to enable the cameras 242 to capture images of one side or multiple sides of the controller 220.

Referring to FIG. 3 together, input means 222 and 224 for generating and outputting a ready signal and a shot signal are disposed on one side of the controller 220. The input means is generally embodied in a button type or a switch type. For the purpose of convenience of explanation, it is hereinafter assumed that the input means is embodied in a button type. A button for input of a ready signal is referred to as a "ready button" 222 and a button for input of a shot signal is referred to as a "shot button" 224.

As illustrated in the drawings, display means such as lamps 2223 and 225 enabling an operator to recognize that the ready button 222 or the shot button 224 is operated is disposed on one side of the controller 220. The controller 220 is also provided with display means 227, 228, and 229 for acquiring X-ray radiation information such as intensity, radiation dose, and radiation period of X-rays radiated from the generator 210 from the generator 210 and displaying the X-ray radiation information.

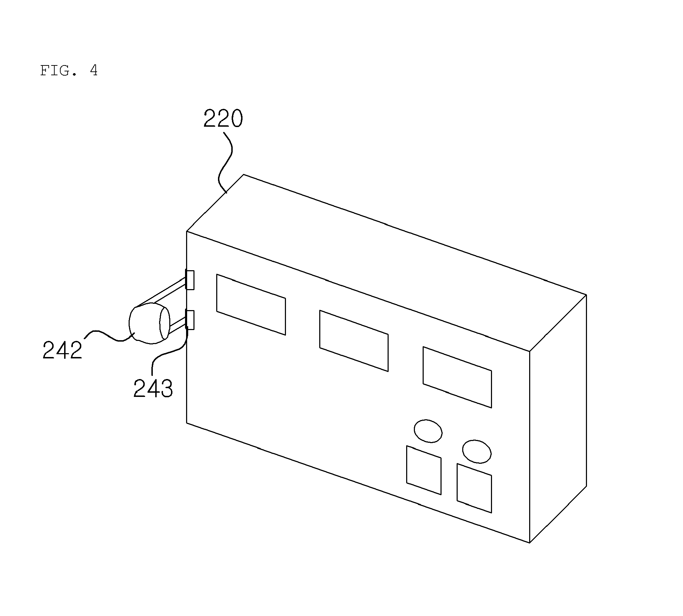

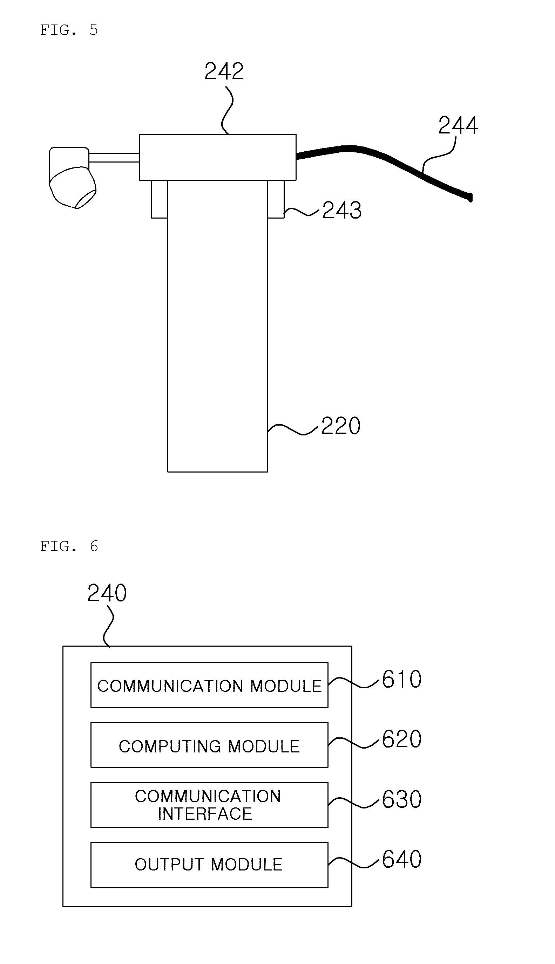

Referring to FIG. 2 again, the camera 242 captures an image of one side of the controller 220 on which the display means 223, 225, 227, 228, and 229 are disposed. In this embodiment, the camera 242 is separated from the controller 220, but the camera 242 may be mounted on or attached to the controller 220 (see FIG. 4 or 5).

The monitoring device 240 according to this embodiment is connected to the camera 242 in a wired manner using a data cable 244 or in a wireless manner and acquires image information captured by the camera 242. The monitoring device 240 can determine whether a control signal such as a ready signal or a shot signal is output from the controller 220 and the type of the control signal, by receiving and analyzing image information, which is captured to include the display means 223, 225, 227, 228, and 229 disposed in the controller 220, from the camera 242 in real time.

That is, the monitoring device 240 can determine whether to output a control signal from the controller 220 by analyzing the received image information to determine whether the lamps 223 and 225 are turned on, and can recognize the type of the control signal by checking which lamp is turned on. Distinction of whether a lamp is turned on or not through image analysis is obviously embodied by image analysis techniques in the related art. For example, by comparing an image captured in a state in which a lamp is turned off and an image captured in a state in which the lamp is turned on, it can be determined whether the lamp is turned on.

In other words, the monitoring device 240 can recognize that the controller 220 transmits the ready signal to the generator 210 when it is determined as the analysis result of the image information received from the camera 242 that the ready lamp 223 is turned on, and can recognize that the controller 220 transmits the shot signal to the generator 210 when it is determined that the shot lamp 225 is turned on.

The monitoring device 240 extracts X-ray radiation information of the display means of the controller 220 from the image information captured by the camera 242. That is, the monitoring device 240 extracts the X-ray radiation information such as intensity, radiation dose, and radiation period of X-rays which is displayed by the display means of the controller 220 included in the captured image information using a character (including numerals) recognition technique based on image analysis.

The monitoring device 240 may output the X-ray radiation information and/or information on the control signal of the controller 220 so as to be recognized by a user through a screen output device (such as an LCD) or a print output device (such as a printer) disposed therein or connected thereto. In the embodiment illustrated in FIG. 2, the monitoring device 240 is embodied by a general computer, but may not be a computer. The monitoring device 240 can be embodied by all types of computing devices that can analyze image information received from the camera 242 and output the analysis result. In this case, the monitoring device 240 can transmit the analyzed control signal and X-ray radiation information to a manager computer and/or a detector 230 connected thereto.

The monitoring device 240 according to this embodiment provides the control signal of the controller 220 to the X-ray detector 230 connected thereto via a communication cable 260. Thereafter, the X-ray detector 230 forms an X-ray image based on X-rays radiated from the generator 210 and transmits the formed X-ray image to the monitoring device 240.

Unlike in the related art in which a cable 22 has to be used for the X-ray detector 230 to directly receive the control signal from the controller 220, since the monitoring device 240 according to this embodiment transmits information on the control signal of the controller 220 to the X-ray detector 230, it is not necessary to connect the X-ray detector 230 and the controller 220 via the cable 22 and it is thus possible to improve convenience of installation of the DR system. Since the communication cable 260 for transmitting an X-ray image is present between the monitoring device 240 and the X-ray detector 230, the information on the control signal can be transmitted and received via the communication cable 260 and an additional cable does not need to be installed, thereby improving convenience of application.

The monitoring device 240 can also provide the X-ray radiation information acquired using the image of the controller 220 captured by the camera 242 to the X-ray detector 230 if necessary.

The camera 242 according to this embodiment can be connected to the monitoring device 240 in a wired manner as illustrated in the drawing, or both may be connected in a wireless manner using a local area network technique such as Zigbee or Bluetooth to transmit the image of the controller 220.

Particularly, the camera 242 may further include a connector 243 enabling attachment to or detachment from one side of the controller 220.

FIGS. 4 and 5 are diagrams illustrating the camera 242 attached to one side of the controller using connectors according to embodiments of the present invention.

Referring to FIG. 4, the camera 242 can be attached to one side of the controller 220 via the connector 243, and captures an image including the display means 223, 225, 227, 228, and 229 of the controller 220 in the attached state.

Referring to FIG. 5 illustrating the shape of the connector 243, the connector 243 for fixing the camera to one side of a side surface of the controller 220 in a sliding structure using a spring or the like may be disposed in the camera 242. The sliding structure of the connector 243 is a structure widely used in an electronic device cradle of a navigation device or the like and is obvious to those skilled in the art. This structure is only an example, and the camera 242 can be attached to the controller 220 in an insertion manner or can be attached to and detached from one side of the controller 220 in various manners using an adhesive.

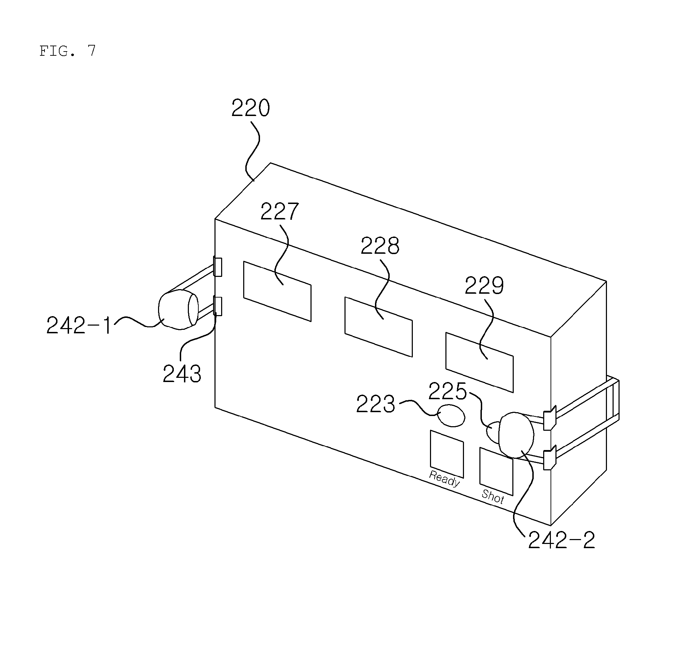

FIG. 7 is a diagram illustrating an example in which plural cameras capturing an image of the controller 220 are provided according to another embodiment of the present invention.

Referring to FIG. 7, for the purpose of more accurate image analysis of the monitoring device 240, plural cameras 242-1 and 242-2 capture an image of the controller 220 and transmit the captured images to the monitoring device 240.

According to this embodiment, the cameras may acquire different images. For example, the first camera 242-1 may acquire an image centered on the lamps 223 and 225 of the controller 220 for the purpose of analysis of the control signal and the second camera 242-2 may acquire an image centered on the display means 227, 228, and 229 of the controller 220 for the purpose of analysis of the radiation information.

The configuration of the monitoring device will be described below.

FIG. 6 is a block diagram illustrating the configuration of the monitoring device according to an embodiment of the present invention.

Referring to FIG. 6, the monitoring device includes a communication module 610, a computing module 620, a communication interface 630, and an output module 640.

The communication module 610 is communication means for receiving image information from the camera 242, and can be embodied in a wired communication manner using a cable and in a wireless communication manner using a local area network communication module as described above.

The computing module 620 serves to analyze image information received from the camera 242 and extracts the control signal and the X-ray radiation information of the controller 220 from the image information.

The communication interface 630 is connected to the communication cable 260 for communication with the X-ray detector 230 and can be embodied, for example, in the form of a port with a structure into which an end of the communication cable 260 is inserted.

The output module 640 serves to output the control signal and the X-ray radiation information of the controller 220 output from the computing module 620 as described above. Since the output module 640 can output information to other devices, the output module 640 may be a communication interface with other devices. Alternatively, the output module 640 also serves to output information in a screen output manner or a print output manner and thus may be a display device such as an LCD or a printing device such as a printer.

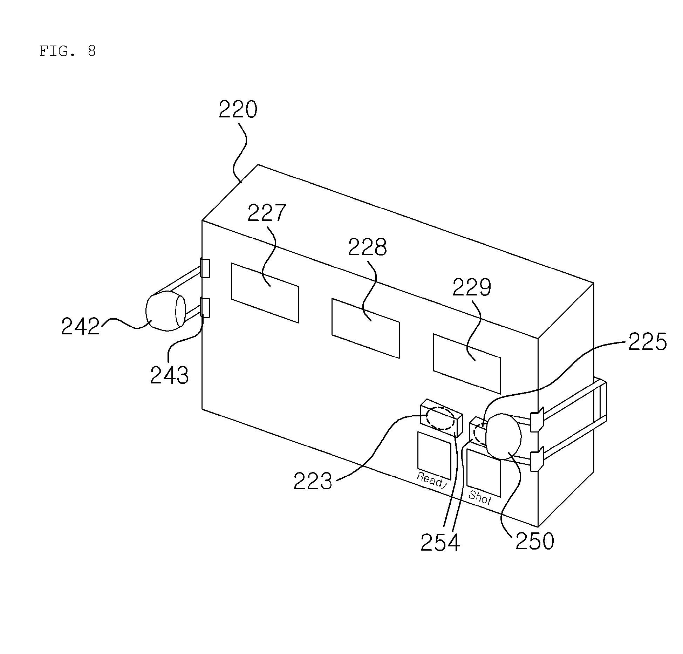

FIG. 8 is a diagram illustrating an example in which sensors 250 and 252 sensing the controller 220 are provided according to another embodiment of the present invention.

In order to recognize the control signal output from the controller 220 to the generator 210, an optical sensor 250 may be used instead of the cameras 242-1 and 242-2. Referring to FIG. 8, the optical sensor 250 is disposed over the lamps 223 and 225, and the optical sensor 250 senses light emitted from the lamps 223 and 225 and transmits the sensing result to the monitoring device 240. The monitoring device 240 can analyze the information output from the optical sensor 250 and recognize the control signal output from the controller 220 to the generator 210.

A cover 254 is disposed on each lamp 223 or 225. The cover 254 can be formed of a material which prevents external light from passing to the inside and outputs internal light (that is, light emitted from the lamps 223 and 225) to the outside. An example of such a material is reflective glass, but is not limited to the reflective glass. By shielding the lamps 223 and 225 using the cover 254 formed of reflective glass, the optical sensor 250 can be prevented from not receiving light emitted from the lamps 223 and 225 due to external light.

The optical sensor 250 is disposed outside the cover 254 as illustrated in FIG. 8, but may be disposed inside the cover 254. In this way, by disposing the optical sensor 250 inside the cover 254 formed of reflective glass or the like, the optical sensor 250 can accurately sense light emitted from the lamps 223 and 225 without being affected by external light.

While the invention is described above with reference to the embodiment, it will be understood by those skilled in the art that the invention can be modified and changed in various forms without departing from the spirit and scope of the invention described in the appended claims.

## Claims

1. A digital radiography system comprising:
an X-ray detector that receives X-rays radiated from a generator of an X-ray system and that forms an X-ray image;
a camera that captures an image of one side surface of a controller outputting a ready signal and a shot signal to control operation of the generator; and
a monitoring device that analyzes image information transmitted from the camera to recognize a control signal output from the controller to the generator and that provides information on the control signal to the X-ray detector.

2. The digital radiography system according to claim 1, wherein the controller includes display means for notifying that the ready signal or the shot signal is output, and
wherein the monitoring device recognizes the ready signal or the shot signal using the image information captured to include the display means in an image.

3. The digital radiography system according to claim 2, wherein the display means of the controller outputs information on at least one of intensity, radiation dose, and radiation period of X-rays radiated from the generator, and
wherein the monitoring device recognizes and outputs the information on at least one of intensity, radiation dose, and radiation period of the X-rays using the image information captured to include the display means in the image.

4. The digital radiography system according to claim 1, wherein the camera includes a connector which is detachably attached to the controller.

5. A digital radiography system comprising:
an X-ray detector that receives X-rays radiated from a generator of an X-ray system and that forms an X-ray image;
an optical sensor that receives light emitted from a controller outputting a ready signal and a shot signal to control operation of the generator; and
a monitoring device that analyzes information transmitted from the optical sensor to recognize a control signal output from the controller to the generator and that provides information on the control signal to the X-ray detector.

6. An X-ray monitoring device comprising:
a communication module that receives image information from a camera module capturing an image of one side of a controller which controls driving of a generator radiating X-rays to an X-ray detector forming an X-ray image;
a computing module that analyzes the image information to recognize a control signal based on a ready signal or a shot signal output from the controller to the generator; and
a communication interface that is connected to a communication cable for transmitting information on the control signal to the X-ray detector.

7. The X-ray monitoring device according to claim 6, wherein the controller includes display means for notifying that the ready signal or the shot signal is output, and
wherein the computing module recognizes the ready signal or the shot signal using the image information captured to include the display means in an image.

8. The X-ray monitoring device according to claim 7, wherein the display means of the controller outputs information on at least one of intensity, radiation dose, and radiation period of X-rays radiated from the generator, and
wherein the computing module recognizes and outputs the information on at least one of intensity, radiation dose, and radiation period of the X-rays using the image information captured to include the display means in the image.
